Europäisches Patentamt

European Patent Office

Office européen des brevets

(19)

(11) EP 0 967 197 A1

(12) DEMANDE DE BREVET EUROPEEN

(43) Date de publication:
29.12.1999 Bulletin 1999/52

(51) Int Cl.$^6$: C07C 67/03, C07C 69/52

(21) Numéro de dépôt: 99390011.7

(22) Date de dépôt: 21.06.1999

(84) Etats contractants désignés:
AT BE CH CY DE DK ES FI FR GB GR IE IT LI LU
MC NL PT SE
Etats d'extension désignés:
AL LT LV MK RO SI

(30) Priorité: 25.06.1998 FR 9808062

(71) Demandeur: Toulousaine de Recherche et de
Developpement "T.R.D."
31190 Auterive (FR)

(72) Inventeurs:
• Mouloungui, Zéphirin
  31000 Toulouse (FR)

• Lacaze-Dufaure, Corinne
  31400 Toulouse (FR)
• Gaset. Antoine
  31000 Toulouse (FR)
• Rigal, Luc
  31240 Saint-Jean (FR)

(74) Mandataire:
Cabinet BARRE LAFORGUE & associés
95, rue des Amidonniers
31000 Toulouse (FR)

(54) **Procédé de fabrication d'esters d'alkyles par transesterification ou alcoolyse**

(57)    L'invention concerne un procédé de fabrication d'un ester d'alkyle, du type dans lequel on fait réagir un ester d'un alkyle différent et un alcool en présence d'un catalyseur acide à une température comprise entre 50°C et 250°C en vue de réaliser un échange de radicaux alkyles par une réaction de transestérification ou d'alcoolyse. Le procédé conforme à l'invention est caractérisé en ce qu'on réalise la réaction en milieu mixte hydrophobe/hydrophile en présence d'un catalyseur acide comprenant essentiellement de l'acide phosphorique ou un sel de cet acide, on sépare par démixion la phase alcool hydrophile et la phase ester hydrophobe, et on sépare le cas échéant dans la phase ester l'ester d'alkyle résiduel et l'ester d'alkyle obtenu.

**Description**

L'invention concerne un procédé de fabrication d'esters d'alkyles à partir d'un ester d'alkyle différent par une réaction de transestérification ou d'alcoolyse permettant un échange des radicaux alkyles. L'invention vise, dans une première gamme d'esters, à fabriquer des esters d'acides gras et d'alkyles par transestérification au moyen d'un alcool d'une huile essentiellement constituée de triglycérides, en particulier huile végétale ou animale. (Par "huile", on entend aussi bien les huiles proprement dites qui sont liquides à température ambiante, que les graisses qui se liquéfient à la température de la réaction de transestérification). L'invention vise, dans une seconde gamme d'esters, à fabriquer des esters d'alkyles ciblés, en particulier à radicaux alkyles supérieurs, par une réaction d'alcoolyse d'un ester d'alkyle différent, en particulier ester d'alkyle courant disponible à bas prix sur le marché.

La transestérification des huiles végétales ou animales au moyen d'un alcool est une réaction bien connue pour fabriquer des esters d'acides gras qui ont de très nombreuses applications industrielles. Cette réaction (triglycérides + alcool $\Rightarrow$ esters d'acides gras + glycérol) a fait l'objet de nombreuses études, en particulier pour ce qui concerne le choix des catalyseurs à utiliser ("R. Sridharan et al, transesterification Reactions, J. Scient. Ind. Res Vol 33, avril 1974, pp 178-186 ; brevet FR 2577569...). Cette transestérification et en particulier utilisée pour fabriquer des esters d'acides gras à radical alkyle court (ester méthylique...).

Par ailleurs, la réaction d'alcoolyse est une réaction d'échange entre un ester d'alkyle et un alcool qui conduit à la formation d'un autre ester avec libération d'une molécule d'alcool comme co-produit (ester1 + alcool2 $\Leftrightarrow$ ester2 + alcool1). Cette réaction d'alcoolyse a également fait l'objet de nombreuses études et les catalyseurs utilisés sont similaires à ceux utilisés par les réactions de transestérifications ci-dessus évoquées.

Les catalyseurs basiques (méthylate de sodium, hydroxyde alcalin) sont préférés dans ces types de réactions car ils conduisent à de bonnes cinétiques de réactions (essentiellement dans le cas d'alcools à courte chaîne). Ces catalyseurs sont les plus utilisés mais ils exigent des précautions particulières : huile ou ester de départ parfaitement purifié, caractère strictement anhydre du milieu réactionnel ; en outre, avec ces catalyseurs basiques, on observe une certaine saponification de l'huile ou de l'ester de départ, et/ou des esters formés, et les savons obtenus rendent difficile la séparation des esters obtenus de la phase hydrophile (phase glycérineuse dans le cas de la transestérification des huiles) : cette saponification conduit à une perte notable de matières premières.

Des études ont montré que les catalyseurs acides forts (acide chlorhydrique, acide sulfonique, acide sulfurique) conduisent également à de bonnes cinétiques des réactions de transestérification ou d'alcoolyse ; mais ce type de catalyseur n'est pas utilisé industriellement en raison des défauts spécifiques qu'ils impliquent (corrosion, manipulations difficiles, toxicité) ; de plus, les catalyseurs acides sont peu sélectifs vis-à-vis des réactions de transestérification ou d'alcoolyse et conduisent à des réactions parasites, engendrant des produits de dégradation qui réduisent les rendements et exigent une purification finale complexe des esters obtenus.

Le mécanisme d'intervention de ces catalyseurs acides est lié à la force de l'acide du fait que ce dernier agit comme donneur de protons. Dans ces conditions, aucune étude n'a été faite sur la catalyse de réactions de transestérification ou d'alcoolyse par des acides faibles qui, a priori, sont supposés inadaptés pour agir de façon satisfaisante comme donneur de protons. Il est à noter que pour des réactions proches (réaction d'estérification d'acides organiques), une étude a conclu que les acides faibles (exemple : acide phosphorique) ont une efficacité réduite, et ceci est expliqué en raison de leur caractère faiblement donneur de protons ("Shah Abdul Majid, Comparative Study of Esterifying effects of different catalysts, J. Sci Ind Res. XXIX, n° 4, 1994, p 13-22").

Il est à noter que le brevet US 3.098.093 cite dans des réactions d'alcoolyse l'utilisation d'un certain nombre de catalyseurs basiques ou acides en milieu homogène, notant parmi ceux-ci certains acides faibles ; toutefois, les essais effectués avec des acides faibles dans les conditions de ce brevet confirment l'absence d'efficacité de ce type de catalyseur. Les mêmes commentaires peuvent être formulés au sujet de la demande WO 96/40701 qui vise une réaction de polyestérification en milieu homogène, conduisant à un polyester de polyol.

Dans ces conditions, toutes les réactions de transestérification d'huile ou d'alcoolyse mises en oeuvre industriellement le sont avec des catalyseurs basiques, les huiles ou esters de départ ayant subi préalablement une purification poussée et les conditions opératoires étant drastiques en vue de garantir le caractère anhydre du milieu réactionnel. Il convient de souligner que la cinétique de transestérification ou d'alcoolyse est bonne sous catalyse basique pour des alcools courts (C1 à C4) permettant d'obtenir des esters à chaîne alkyle courte ; par contre, cette cinétique chute considérablement pour des chaînes longues, de sorte qu'il n'existe à l'heure actuelle aucun procédé industriel satisfaisant pour produire des esters d'alkyles à longues chaînes, notamment esters d'acides gras.

La présente invention se propose de fournir un procédé de fabrication d'esters d'alkyles, par transestérification d'huile ou par alcoolyse d'un ester, qui bénéficie d'une sélectivité totale, d'un rendement élevé (par rapport au rendement auquel conduisent les catalyseurs conventionnels) et ne présente pas les défauts des procédés sous catalyse basique ou acide connus.

Un autre objectif est de permettre l'obtention d'esters d'alkyle à longue chaîne, en particulier esters d'acides gras.

Un autre objectif est de fournir un procédé qui soit applicable de façon satisfaisante sur des huiles brutes ou esters dérivés (n'ayant subi aucune purification préalable) susceptibles d'être contaminé par des phospholipides et des acides gras libres.

Un autre objectif est de faciliter la séparation des esters obtenus et de l'alcool formé.

A cet effet, le procédé visé par l'invention pour fabriquer des esters d'alkyle consiste à faire réagir un ester d'un alkyle différent et un alcool en présence d'un catalyseur acide à une température comprise entre 50° C et 250° C en vue de réaliser un échange de radicaux alkyles par une transestérification ou alcoolyse : ester d'alkyle 1 + alcool 2 ⇒ ester d'alkyle 2 + alcool 1. Le procédé conforme à l'invention se caractérise en ce que :

.   on réalise la réaction en milieu mixte hydrophobe/hydrophile en présence d'un catalyseur acide comprenant essentiellement de l'acide phosphorique ou un sel de cet acide,

.   on sépare par démixion la phase alcool hydrophile et la phase ester hydrophobe, et on sépare le cas échéant dans la phase ester l'ester d'alkyle 1 résiduel et l'ester d'alkyle 2 obtenu.

De façon inattendue, les expérimentations ont permis de constater que, en milieu hydrophobe/hydrophile, c'est-à-dire dans un milieu réactionnel dans lequel la phase organique se trouve en présence d'une phase hydrophile, on obtient une transestérification ou une alcoolyse de l'ester de départ en utilisant comme catalyseur un acide faible essentiellement constitué par l'acide phosphorique. La sélectivité de la réaction est très élevée et de 100 % dans les meilleures conditions ; son rendement aux températures habituelles de ces types de réaction (50° - 250° C) est notablement supérieur à celui obtenu avec les catalyseurs classiques. De préférence, la réaction est réalisée à une température inférieure à la température d'ébullition du milieu réactionnel. Ainsi, dans le cas d'une transestérification de triglycérides pour obtenir un ester d'acide gras et d'alkyle, le rendement atteint des valeurs de l'ordre de 80 % à 98 %. Dans le cas d'une alcoolyse (réaction équilibrée), on obtient des taux de conversion généralement supérieurs à 65% en ester à radical alkyle différent de l'ester de départ. Aucune réaction parasite n'est observée et l'on obtient les composés recherchés avec une très bonne pureté. Dans le milieu hydrophile/hydrophobe réactionnel, ces composés sont faciles à séparer par simple décantation à froid de la phase hydrophile, la phase hydrophobe supérieure étant prélevée après séparation des phases. Dans cette phase hydrophobe se trouve l'ester d'alkyle recherché, éventuellement de l'ester d'alkyle de départ et de l'alcool résiduel ; cet alcool est facile le cas échéant à séparer par distillation fractionnée.

Une autre caractéristique remarquable du procédé de l'invention est que l'ester de départ peut être une huile brute (non raffinée) ou un ester d'alkyle provenant d'une huile brute, ou même un broyat de graines oléagineuses.

Dans le procédé de l'invention, il semble que l'acide phosphorique remplisse plusieurs fonctions différentes : fonction de catalyseur par un mécanisme encore inexpliqué (qui ne se résout pas à un simple mécanisme de donneurs de protons), fonction de dégommage consistant à complexer les phospholipides qui se retrouvent à l'état de phospholipides hydratables (forme acide) dans la phase hydrophile, fonction favorisant la décantation du milieu hydrophobe/hydrophile en raison du caractère très hydrophile du catalyseur (effet opposé à celui des savons qui empêchent cette décantation). Il est à noter que la deuxième fonction est en elle-même connue puisque l'acide phosphorique est déjà utilisé pour le prétraitement d'huiles brutes par décomposition des sels non hydratables des phospholipides ("Paul F. Kusy, Transesterification of vegetable oils for fuels, ASAE publication 4-1982 pp 127-137").

Dans le cas où l'ester recherché est à chaîne alkyle courte, la cinétique de la réaction est moins bonne qu'en utilisant un catalyseur basique ou acide fort mais elle est parfaitement acceptable en lipo-chimie industrielle, et le procédé de l'invention constitue un progrès significatif par rapport aux procédés connus compte tenu des avantages considérables qu'il apporte (absence de réactions parasites de dégradation, pas de formation de savons, utilisation de matière première brute, réactifs non agressifs, facilité de séparation). La cinétique de la réaction dans l'invention est peu affectée par la longueur de la chaîne alkylée de l'ester recherchée : dans le cas de chaînes longues, on obtient une cinétique significativement plus importante qu'en utilisant un catalyseur basique.

En particulier, avec des alcools lourds à longue chaîne linéaire ou ramifiée, les essais ont montré que la réaction se produit avec des taux de conversion élevés et une cinétique du même ordre que dans le cas des alcools courts, de sorte que le procédé de l'invention permet de fabriquer non seulement des esters d'alkyle à courte chaîne mais également des esters à longue chaîne alkylée. Lorsqu'on utilise comme réactif un alcool lourd (nombre de carbones supérieur ou égal à 8), la température du milieu réactionnel est avantageusement choisie plus élevée que dans le cas contraire. En pratique, elle pourra avantageusement être ajustée entre 120° C et 170° C selon l'alcool utilisé. Au contraire, si on utilise un alcool court (nombre de carbones inférieur à 8), la température sera avantageusement ajustée dans une plage comprise entre 60° et 120° C. Dans tous les cas, on utilise de préférence comme réactif un mono-alcool.

De préférence, on ajuste la quantité d'alcool par rapport à l'ester réactif pour se placer au dessus de la stoechiométrie de la réaction de transestérification ou d'alcoolyse, en particulier dans une plage 2P - 4P, où P est la quantité d'alcool correspondant à la stoechiométrie de ladite réaction. Il est à noter que cette quantité d'alcool n'est pas critique à condition d'être supérieure à la stoechiométrie ; un excès trop important d'alcool n'apporte généralement aucun avantage et conduit à des volumes inutiles.

Le procédé de l'invention paraît fournir de meilleurs résultats si une quantité d'eau modérée est ajoutée au milieu de façon que la réaction se déroule en milieu hydro-organique. En particulier, le milieu réactionnel est fortement agité pour l'homogénéiser et la présence d'eau favorise sa dispersion sous la forme d'une émulsion fine et stable : une telle émulsion améliore les échanges d'interface et donc la cinétique de la réaction de transestérification ou d'alcoolyse. La température du milieu est prévue inférieure à sa température d'ébullition pour éviter un entraînement de l'eau.

On peut utiliser avantageusement de l'acide phosphorique dans de l'eau de façon à former le milieu hydro-organique précité lors de l'introduction du catalyseur phosphorique. L'acide en milieu aqueux est préférentiellement mélangé au milieu réactionnel en quantité telle que la proportion pondérale d'acide phosphorique par rapport à l'ester d'alkyle de départ soit sensiblement comprise entre 10 % et 50 % et que la proportion pondérale d'eau par rapport audit ester soit sensiblement comprise entre 0,5 % et 5 %. On peut notamment utiliser de l'acide phosphorique à 85 %.

Dans certaines conditions opératoires (en particulier en cas d'excès d'acide phosphorique par rapport à la plage préférentielle sus-indiquée), on peut constater la présence de traces d'acide phosphorique dans la phase hydrophobe prélevée. Cet acide peut, le cas échéant, être séparé de l'ester par passage sur résine adsorbante ou sur gel de silice.

Comme déjà évoqué, dans un premier type d'application, le procédé de l'invention peut être mis en oeuvre pour fabriquer un ester d'acide gras et d'alkyle à partir d'une huile essentiellement constituée de triglycérides. La réaction est alors une réaction de transestérification réalisant l'échange des radicaux alkyles par déplacement du glycérol : triglycérides + alcool $\Rightarrow$ ester d'acides gras + glycérol. L'huile utilisée peut être une huile brute provenant directement de la trituration de graines oléagineuses. Il est également possible de coupler l'opération d'extraction de l'huile à partir des graines et la transestérification. Dans ce cas, le broyat provenant de l'action mécanique de broyage desdites graines (avant extraction de l'huile) est mis en présence des réactifs et catalyseur de transestérification conformément à l'invention de façon que l'huile native soit transformée conformément au procédé de l'invention. Quelque soit l'état du substrat oléophile de départ, la réaction présente un rendement élevé et la majorité des triglycérides est transformée en ester d'acides gras. Le glycérol qui est recueilli après décantation peut être valorisé, soit directement, par exemple comme matière première servant à la fabrication de dérivés du glycérol, soit après élimination des autres constituants de la phase hydrophile (eau, acide phosphorique, phosphatides, alcool réactif résiduel). Une simple distillation fractionnée permet, en cas de besoin, de séparer le glycérol de l'alcool réactif résiduel en vue d'une valorisation dudit glycérol.

Le procédé de l'invention peut également être mis en oeuvre, comme déjà indiqué, pour fabriquer par une réaction d'alcoolyse équilibrée, un ester d'alkyle ciblé possédant en particulier un nombre de carbones égal ou supérieur à 4. On utilise alors comme réactif de départ un ester d'alkyle ayant un radical alkyle à nombre de carbones inférieur à 4 en vue de réaliser un échange des radicaux alkyles avec ceux de l'alcool lourd servant de réactif de départ. On peut ainsi à partir d'esters courts disponibles sur le marché et d'alcools lourds de faible coût, obtenir des esters à longues chaînes alkylées dont la valeur est beaucoup plus élevée. Dans ce cas, après démixion, on sépare dans la phase hydrophobe l'ester d'alkyle à radical supérieur obtenu par distillation fractionnée ou extraction liquide/liquide, dans des conditions propres à éliminer l'ester de départ résiduel. Ce type de séparation jouant sur les différences de température d'ébullition ou de capacité de dissolution est classique en chimie.

Les exemples qui suivent illustrent le procédé de l'invention et ses performances. Dans tous les exemples, la température à laquelle est porté le milieu réactionnel est inférieure à la température d'ébullition dudit milieu et assez éloignée de celle-ci pour éviter tout début d'ébullition.

EXEMPLE 1 : Transestérification d'une huile de tournesol oléique brute

Composés, utilisés et proportions

L'huile utilisée est une huile brute de pression à 99 % en triglycérides. Elle présente un indice d'acide, déterminé selon la norme NF T 60-204, de 1,6 et une teneur en phosphore, déterminé selon la norme NF T 60-227, de 98 mg/kg d'huile. Son taux d'humidité, déterminé selon la méthode Karl-Fisher, et de 0,05%. La composition de ses triglycérides a été déterminée par chromatographie en phase gazeuse après méthylation selon les normes NF T 60-233 et NF T 60-23 :

- 3,2 % d'acide palmitique,
- 2,4 % d'acide stéarique,
- 89,9 % d'acide oléique,
- 4,5 % d'acide linoléique.

L'alcool utilisé est le 2-éthyl hexanol. Il est utilisé à une quantité correspondant à 1.P (où P représente la quantité d'alcool correspondant à la stoechiométrie de la réaction) soit à un rapport massique 2-éthyl hexanol/huile de 44 %.

Le catalyseur utilisé est l'acide phosphorique à 85 % dans l'eau. Il est introduit à un rapport massique H3PO4 pur/huile de 16 %. On introduit ainsi 2,85 % d'eau dans le milieu (en poids par rapport à l'huile).

Mise en oeuvre

a/ Une masse d'huile de 8,80 g (soit 0,01 mol de triglycérides) est introduite dans un tricol ayant une contenance de 100 ml. L'alcool puis le catalyseur sont ensuite ajoutés dans les proportions précédemment indiquées.

Le tricol est équipé d'une arrivée d'azote, d'un réfrigérant et d'une canne d'agitation mécanique. Le temps de réaction est décompté à partir du moment où l'on plonge le réacteur dans un bain d'huile préchauffé. Le milieu réactionnel de type mixte hydrophobe/hydrophile est chauffé à 130° C et agité à 250 tr/min pour l'homogénéiser et le disposer sous la forme d'une émulsion fine et stable. Le temps de réaction est de 90 minutes.

b/ A l'issue de la réaction, le ballon est plongé 20 min dans un bain d'eau à 5° C (eau+glace) pour stopper la réaction. Il y a immédiatement décantation de 2 phases.

c/ La phase supérieure hydrophobe est diluée par ajout de 40 ml d'hexane, séchée sur $MgSO_4$ puis évaporée. Elle est analysée par Chromatographie sur Couche Mince couplée à une Détection par Ionisation de Flamme. Les éluants utilisés et les temps de migration sont :

- 25 min de développement dans le premier éluant (hexane/diéthyléther/acide formique 85/15/0,04 en ml).
- 20 min de développement dans le second éluant (hexane/diéthyléter/acide formique 65/35/0,04 en ml).

Résultats obtenus

- La phase inférieure est constituée d'eau et de glycérol.

La composition massique de la phase hydrophobe contenant les esters est déterminée par étalonnage interne (étalon interne : cholestérol).

• On calcule le rendement en esters de la réaction :

$$R(\%) = (\text{masse d'esters formés/masse potentielle d'esters}) * 100$$

Masse potentielle d'esters : masse d'esters formés si tous les triglycérides présents dans l'huile étaient transestérifiés.

- La sélectivité de la réaction est définie par la relation suivante :

$$S(\%) = (\text{masse d'esters formés}) / (\text{masse d'esters} + \text{masse de monoglycérides} + \text{masse de diglycérides})$$

- Les conditions opératoires mises en jeu dans cet exemple n° 1 conduisent à :

* un rendement en esters de 2-éthyl hexyle de 54,0 %
* une sélectivité de la réaction de 100 %.

Dans cet exemple, la quantité d'alcool mis en oeuvre qui est de 1.P, n'est pas suffisante pour permettre une réaction totale des triglycérides.

Les exemples 2 à 9 qui suivent présentent des mises en oeuvre analogues avec modification de certains paramètres.

EXEMPLE 2 :

L'huile utilisée est la même que celle de l'exemple n° 1.

L'alcool utilisé est le 2-éthyl hexanol. Il est utilisé à une quantité correspondant à 5.P (où P représente la quantité d'alcool correspondant à la stoechiométrie de la réaction) soit à un rapport massique 2-éthyl hexanol/huile de 222 %.

Le catalyseur utilisé est l'acide phosphorique à 85 % dans l'eau. Il est introduit à un rapport massique H3PO4 pur/huile de 16 %. On introduit ainsi 2,85 % d'eau dans le milieu (en poids par rapport à l'huile).

Une masse d'huile de 8,80 g (soit 0,01 mol de triglycérides) est introduite dans un tricol de 100 ml. L'alcool puis le catalyseur sont ensuite ajoutés.

Le dispositif expérimental et la mise en oeuvre sont les mêmes qu'à l'exemple n° 1. Le milieu réactionnel est chauffé à 130° C et agité à 250 tr/min. Le temps de réaction est de 90 minutes.

A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur $MgSO_4$ puis évaporée. Elle est analysée par Chromatographie sur Couche Mince couplée à une Détection par Ionisation de Flamme (conditions d'analyse décrites dans l'exemple n° 1).

On calcule le rendement en esters et la sélectivité de la réaction selon les relations précitées. Les conditions opératoires mises en jeu dans cet exemple n° 2 conduisent à :

- un rendement en esters de 2-éthyl hexyle de 38,9 %,
- une sélectivité de la réaction de 100 %.

Dans cet exemple, on assiste à une diminution du rendement en esters liée à une dilution excessive du milieu réactionnel.

Ainsi, on utilisera de préférence l'alcool à une proportion supérieure de la stoechiométrie P, en pratique entre 2 P et 4 P, afin d'optimiser le rendement.

EXEMPLE 3 :

L'huile utilisée est la même que celle de l'exemple n° 1.

L'alcool utilisé est le 2-éthyl hexanol. Il est utilisé à une quantité correspondant à 3.P (où P représente la quantité d'alcool correspondant à la stoechiométrie de la réaction) soit à un rapport massique 2-éthyl hexanol/huile de 132 %.

Le catalyseur utilisé est l'acide phosphorique à 85 % dans l'eau. Il est introduit à un rapport massique H3PO4 pur/huile de 10 %. On introduit ainsi 1,75 % d'eau dans le milieu (en poids par rapport à l'huile).

Une masse d'huile de 8,80 g (soit 0,01 mol de triglycérides) est introduite dans un tricol de 100 ml. L'alcool puis le catalyseur sont ensuite ajoutés.

Le dispositif expérimental et la mise en oeuvre sont les mêmes qu'à l'exemple n° 1. Le milieu réactionnel est chauffé à 130° C et agité à 250 tr/min. Le temps de réaction est de 90 minutes.

A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur $MgSO_4$ puis évaporée. Elle est analysée par chromatographie sur Couche Mince couplée à une Détection par Ionisation de Flamme (conditions d'analyse décrites dans l'exemple n° 1).

On calcule le rendement en esters et la sélectivité de la réaction selon les relations précitées. Les conditions opératoires mises en jeu dans cet exemple n° 3 conduisent à :

- un rendement en esters de 2-éthyl hexyle de 71,3 %,
- une sélectivité de la réaction de 100 %.

On note dans cet exemple que la quantité d'acide phosphorique utilisée représente une limite inférieure de réactivité.

Ainsi on utilisera de préférence l'acide phosphorique à une quantité telle que la proportion pondérale d'acide phosphorique par rapport à l'ester soit comprise entre 10 % et 50 %.

EXEMPLE 4 :

L'huile utilisée est la même que celle de l'exemple n° 1.

L'alcool utilisé est le 2-éthyl hexanol. Il est utilisé à une quantité correspondant à 3.P (où P représente la quantité d'alcool correspondant à la stoechiométrie de la réaction) soit à un rapport massique 2-éthyl hexanol/huile de 132 %.

Le catalyseur utilisé est l'acide phosphorique à 85 % dans l'eau. Il est introduit à un rapport massique H3PO4 pur/huile de 16 %. On introduit ainsi 2,85 % d'eau dans le milieu (en poids par rapport à l'huile).

Une masse d'huile de 8,80 g (soit 0,01 mol de triglycérides) est introduite dans un tricol de 100 ml. L'alcool puis le catalyseur sont ensuite ajoutés.

Le dispositif expérimental et la mise en oeuvre sont similaires à l'exemple n° 1. Le milieu réactionnel est chauffé à 130° C et agité à 250 tr/min. Le temps de réaction est de 240 minutes.

A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur MgSO$_4$ puis évaporée. Elle est analysée par Chromatographie sur Couche Mince couplée à une Détection par Ionisation de Flamme (conditions d'analyse décrites dans l'exemple n° 1 ci-dessus).

On calcule le rendement en esters et la sélectivité de la réaction selon les relations précitées. Les conditions opératoires mises en jeu dans cet exemple n° 4 conduisent à :

- un rendement en esters de 2-éthyl hexyle de 98,0 %,
- une sélectivité de la réaction de 100 %.

Les conditions optimales sont mises en oeuvre dans cet exemple.

EXEMPLE 5 :

L'huile utilisée est la même que celle de l'exemple n° 1.

L'alcool utilisé est le 2-éthyl hexanol. Il est utilisé à une quantité correspondant à 3.P (où P représente la quantité d'alcool correspondant à la stoechiométrie de la réaction) soit à un rapport massique 2-éthyl hexanol/huile de 132 %.

Le catalyseur utilisé est l'acide phosphorique à 85 % dans l'eau. Il est introduit à un rapport massique H3PO4 pur/huile de 3,33 %. On introduit ainsi 0,59 % d'eau dans le milieu (en poids par rapport à l'huile).

Une masse d'huile de 8,80 g (soit 0,01 mol de triglycérides) est introduite dans un tricol de 100 ml. L'alcool puis le catalyseur sont ensuite ajoutés.

Le dispositif expérimental est décrit dans l'exemple n° 1 ci-dessus. Le milieu réactionnel est chauffé à 160° C et agité à 250 tr/min pour homogénéiser le milieu réactionnel. Le temps de réaction est de 360 minutes.

A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur MgSO$_4$ puis évaporée. Elle est analysée par Chromatographie sur Couche Mince couplée à une Détection par Ionisation de Flamme (conditions d'analyse décrites dans l'exemple n° 1 ci-dessus).

On calcule le rendement en esters et la sélectivité de la réaction selon les relations précitées. Les conditions opératoires mises en jeu dans cet exemple n° 5 conduisent à :

- un rendement en esters de 2-éthyl hexyle de 97,0 %,
- une sélectivité de la réaction de 100 %.

On note une réaction sensiblement totale des triglycérides. L'influence défavorable de la faible quantité d'acide phosphorique mis en jeu est corrigée par l'apport thermique élevé et le contrôle de la teneur en eau du milieu réactionnel.

EXEMPLE 6 :

L'huile utilisée est la même que celle de l'exemple n° 1.

L'alcool utilisé est le 2-éthyl hexanol. Il est utilisé à une quantité de 2.P (où P représente la quantité d'alcool correspondant à la stoechiométrie de la réaction) soit à un rapport massique 2-éthyl hexanol/huile de 88 %.

Le catalyseur utilisé est l'acide phosphorique à 85 % dans l'eau. Il est introduit à un rapport massique H3PO4 pur/huile de 11 %. On introduit ainsi 1,94 % d'eau dans le milieu (en poids par rapport à l'huile).

Une masse d'huile de 200 g (soit 0,23 mol de triglycérides) est introduite dans un réacteur à 3 cols de 1,5 ℓ. L'alcool puis le catalyseur sont ensuite ajoutés.

Le réacteur est équipé d'une arrivée d'azote, d'un réfrigérant et d'une canne d'agitation mécanique. Le milieu réactionnel est chauffé à 130° C sous balayage d'azote et agité à 500 tr/min pour homogénéiser le milieu réactionnel. Le temps de réaction est de 7 heures.

A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur $MgSO_4$ puis évaporée. Elle est analysée par Chromatographie sur Couche Mince couplée à une Détection par Ionisation de Flamme (conditions d'analyse décrites dans l'exemple n° 1).

On calcule le rendement en esters et la sélectivité de la réaction selon les relations précitées. Les conditions opératoires mises en jeu dans cet exemple n° 6 conduisent à :

- un rendement en esters de 2-éthyl hexyle de 86,2 %,
- une sélectivité de la réaction de 100 %.

Dans cet exemple, la quantité d'acide phosphorique faible qui représente la borne inférieure du domaine est corrigée par la durée de réaction qui est allongée.

EXEMPLE 7 :

L'huile utilisée est la même que celle de l'exemple n° 1.

L'alcool utilisé est le 2-éthyl hexanol. Il est utilisé à une quantité correspondant à 5.P (où P représente la quantité d'alcool correspondant à la stoechiométrie de la réaction) soit à un rapport massique 2-éthyl hexanol/huile de 222 %.

Le catalyseur utilisé est l'acide phosphorique pur à 99 %. Il est introduit à un rapport massique H3PO4 pur/huile de 16 %. On introduit ainsi 0,16 % d'eau dans le milieu (en poids par rapport à l'huile).

Une masse d'huile de 8,80 g (soit 0,01 mol de triglycérides) est introduite dans un tricol de 100 ml. L'alcool puis le catalyseur sont ensuite ajoutés.

Le dispositif expérimental et la mise en oeuvre sont identiques à ceux de l'exemple n° 1. Le milieu réactionnel est chauffé à 130° C et agité à 250 tr/min. Le temps de réaction est de 90 minutes.

A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur $MgSO_4$ puis évaporée. Elle est analysée par Chromatographie sur Couche Mince couplée à une Détection par Ionisation de Flamme (conditions d'analyse décrites dans l'exemple n° 1).

On calcule le rendement en esters et la sélectivité de la réaction selon les relations précitées. Les conditions opératoires mises en jeu dans cet exemple n° 7 conduisent à :

- un rendement en esters de 2-éthyl hexyle de 60,0 %,
- une sélectivité de la réaction de 100 %.

L'effet néfaste de la dilution est dans cet exemple corrigé par le contrôle de la teneur en eau du milieu. Ainsi, on se placera de préférence à une teneur en eau du milieu comprise entre 0,1 % et 15 %.

Les exemples 8 et 9 qui suivent sont mis en oeuvre avec des alcools différents.

EXEMPLE 8 : Utilisation de l'octanol comme réactif

L'huile utilisée est la même que celle de l'exemple n° 1.

L'alcool utilisé est l'octanol. Il est utilisé à une quantité correspondant à 2.P (où P représente la quantité d'alcool correspondant à la stoechiométrie de la réaction) soit à un rapport massique 2-éthyl hexanol/huile de 88 %.

Le catalyseur utilisé est l'acide phosphorique à 85 % dans l'eau. Il est introduit à un rapport massique H3PO4/huile de 16 %. On introduit ainsi 2,85 % d'eau dans le milieu (en poids par rapport à l'huile).

Une masse d'huile de 8,80 g (soit 0,01 mol de triglycérides) est introduite dans un tricol de 100 ml. L'alcool puis le catalyseur sont ensuite ajoutés.

Le dispositif expérimental et la mise en oeuvre sont identiques à ceux de l'exemple n° 1. Le milieu réactionnel est chauffé à 130° C et agité à 250 tr/min. Le temps de réaction est de 180 minutes.

A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur $MgSO_4$ puis évaporée. Elle est analysée par Chromatographie sur Couche Mince couplée à une Détection par Ionisation de Flamme (conditions d'analyse décrites dans l'exemple n° 1 ci-dessus). Pour des raisons pratiques, dans les essais mettant en oeuvre des alcool différents, on détermine par étalonnage interne (étalon interne : cholestérol) la teneur en triglycérides résiduels.

On calcule le taux de conversion des triglycérides selon la relation :

$$T(\%) = ((\text{masse d'huile consommée})/(\text{masse d'huile de départ})) * 100$$

Les conditions opératoires mises en jeu dans cet exemple n° 8 conduisent à un taux de conversion des triglycérides de 96,6 %.

EXEMPLE 9 : Utilisation de l'alcool oléique

L'huile utilisée est la même que celle de l'exemple n° 1.

L'alcool utilisé est l'alcool oléique. Il est utilisé à une quantité correspondant à 2.P (où P représente la quantité d'alcool correspondant à la stoechiométrie de la réaction).

Le catalyseur utilisé est l'acide phosphorique à 85 % dans l'eau. Il est introduit à un rapport massique H3PO4 pur/huile de 16 %. On introduit ainsi 2,85 % d'eau dans le milieu (en poids par rapport à l'huile).

Une masse d'huile de 8,80 g (soit 0,01 mol de triglycérides) est introduite dans un tricol de 100 ml. L'alcool puis le catalyseur sont ensuite ajoutés.

Le dispositif expérimental et la mise en oeuvre sont identiques à ceux décrits à l'exemple n° 1. Le milieu réactionnel est chauffé à 130° C et agité à 250 tr/min. Le temps de réaction est de 180 minutes. A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur $MgSO_4$ puis évaporée. Elle est analysée par Chromatographie sur Couche Mince couplée à la Détection par Ionisation de Flamme (conditions d'analyse décrites dans l'exemple n° 1 ci-dessus). On détermine par étalonnage interne (étalon interne : cholestérol) sa teneur en triglycérides résiduels.

On calcule le taux de conversion des triglycérides selon la relation :

$$T(\%) = ((\text{masse d'huile consommée})/(\text{masse d'huile de départ}))*100$$

Les conditions opératoires mises en jeu dans cet exemple n° 9 conduisent à un taux de conversion des triglycérides de 96,6 %.

EXEMPLE 10 : Alcoolyse d'un ester issu d'une huile de tournesol oléique

Composés, utilisés et proportions

On réalise dans cet exemple l'alcoolyse de l'oléate de méthyle de tournesol, issu d'une transestérification d'huile de tournesol oléique préalable selon les procédés industriels classiques à la soude. L'ester méthylique utilisé est composé à 80 % d'oléate de méthyle.

L'alcool utilisé est le 2-éthyl hexanol. Il est utilisé à une quantité correspondant à 2.P (où P représente la quantité d'alcool correspondant à la stoechiométrie de la réaction) soit à un rapport massique 2-éthyl hexanol/huile de 88 %.

Le catalyseur utilisé est l'acide phosphorique à 85 % dans l'eau. Il est introduit à un rapport massique H3PO4 pur/ester méthylique de 49 %. On introduit ainsi 8,65 % d'eau dans le milieu (en poids par rapport à l'huile).

Une masse d'ester méthylique de 8,88 g (soit 0,03 mol de triglycérides) est introduite dans un tricol de 100 ml. L'alcool puis le catalyseur sont ensuite ajoutés.

Le dispositif expérimental et la mise en oeuvre sont identiques à ceux de l'exemple n° 1. Le milieu réactionnel est chauffé à 130° C et agité à 250 tr/min pour l'homogénéiser et le disposer sous forme d'une émulsion fine et stable. Le temps de réaction est de 120 minutes.

A l'issue de la réaction, le ballon est plongé 20 min dans un bain d'eau à 5° C (eau+glace) pour stopper la réaction. Il y a immédiatement décantation de 2 phases.

La phase supérieure hydrophobe est diluée par ajout de 40 ml d'hexane, séchée sur $MgSO_4$ puis évaporée. Elle est analysée par Chromatographie en phase gazeuse. Pour des raisons pratiques, dans cet exemple de mise en oeuvre de l'alcoolyse d'un ester issu d'une huile de tournesol oléique, on détermine par étalonnage interne (étalon interne : dodécanol) la teneur en oléate de méthyle n'ayant pas réagi.

On calcule ainsi le taux de conversion de l'oléate de méthyle par la relation :

$$T(\%) = 100*(\text{masse d'oléate de méthyle résiduelle/masse d'oléate de méthyle de départ})$$

Les conditions opératoires mises en jeu dans cet exemple n° 10 conduisent à un taux de conversion de l'oléate de méthyle de 70 %.

EXEMPLE 11 : Transestérification d'un broyat de graines de tournesol oléique

Composés utilisés et proportions

Les graines de tournesol utilisées sont des graines à haute teneur en acide oléique (variété "Olbaril"). Elles présentent une teneur en huile de 46,0 % par rapport à la matière sèche et une humidité de 4,15 %.
L'alcool utilisé est le 2-éthyl hexanol. Il est utilisé à une quantité qui correspond à 32.P (où P représente la quantité d'alcool correspondant à la stoechiométrie de la réaction de transestérification des triglycérides présents dans les graines) soit à un rapport massique 2-éthyl hexanol/huile de 1410 %.
Le catalyseur utilisé est l'acide phosphorique à 85 % dans l'eau. Il est introduit à un rapport massique H3PO4 pur/huile de 83,4 %. On introduit ainsi dans le milieu 14,7 % d'eau (en poids par rapport à l'huile).

Mise en oeuvre

a/ Une masse de 20 g de graines (soit 0,01 mol de triglycérides) sont finement broyées à l'aide d'un mixeur "Magimix".
Le broyat est introduit dans un tricol de 500 ml. L'alcool est ensuite ajouté et on fait macérer les graines 15 minutes sous agitation. Le catalyseur est ensuite introduit dans les proportions précédemment indiquées.
Le tricol est équipé d'une arrivée d'azote, d'un réfrigérant et d'une canne d'agitation mécanique. Le temps de réaction est décompté à partir du moment où l'on plonge le réacteur dans un bain d'huile préchauffé. Le milieu réactionnel de type mixte hydrophobe/hydrophile est chauffé à 130° C et agité à 250 tr/min pour l'homogénéiser et le disposer sous la forme d'une émulsion fine et stable. Le temps de réaction est de 4 heures.
b/ A l'issue de la réaction, le ballon est refroidi 20 min dans un bain d'eau à 5° C (eau+glace) pour stopper la réaction.
c/ La phase solide constituée des fibres et protéines des graines est séparée de la phase liquide hydrophobe par centrifugation (2500 tr/min - 15 min).
d/ La phase supérieure hydrophobe est diluée par ajout de 40 ml d'hexane, séchée sur $MgSO_4$ puis évaporée. Elle est analysée par Chromatographie sur Couche Mince couplée à une Détection par Ionisation de Flamme (conditions d'analyse décrites dans l'exemple n° 1).
e/ On calcule le rendement en esters selon la relation précitée. Les conditions opératoires mises en jeu dans cet exemple n° 11 conduisent à un rendement en esters de 60,6 %.

Dans cet exemple, une quantité d'alcool élevée est nécessaire pour permettre l'imprégnation des graines de tournesol par l'alcool et l'agitation du milieu. Une quantité d'acide phosphorique élevée est également nécessaire car la matrice fibreuse hydrophile de la graine adsorbe une quantité non négligeable de l'acide phosphorique. Celui-ci n'est donc plus disponible pour la catalyse de la réaction de transestérification des triglycérides.

EXEMPLE 12 :

Cet exemple permet d'apprécier l'effet favorable de la diminution de la dilution du milieu.
Les graines utilisées sont des graines à haute teneur en acide oléique. Elles présentent une teneur en huile de 43,0 % par rapport à la matière sèche et une humidité de 4,33 %.
L'alcool utilisé est le 2-éthyl hexanol. Il est utilisé à une quantité qui correspond à 21.P (où P représente la quantité d'alcool correspondant à la stoechiométrie de la réaction de transestérification des triglycérides présents dans les graines) soit à un rapport massique 2-éthyl hexanol/huile de 1009 %.
Le catalyseur utilisé est l'acide phosphorique à 85 % dans l'eau. Il est introduit à un rapport massique H3PO4 pur/huile de 83,4 %. On introduit ainsi dans le milieu 14,7 % d'eau (en poids par rapport à l'huile).
Le dispositif expérimental et la mise en oeuvre sont les mêmes qu'à l'exemple n° 11. Le milieu réactionnel est chauffé à 130° C et agité à 250 tr/min. Le temps de réaction est de 4 heures.
A l'issue de la réaction, le ballon est refroidi 20 min dans un bain d'eau à 5° C (eau+glace) pour stopper la réaction.

La phase solide constituée des fibres et protéines des graines est séparée de la phase liquide hydrophobe par centrifugation (2500 tr/min - 15 min).

La phase supérieure hydrophobe est diluée par ajout de 40ml d'hexane, séchée sur $MgSO_4$ puis évaporée. Elle est analysée par Chromatographie sur Couche Mince couplée à une Détection par Ionisation de Flamme (conditions d'analyse décrites dans l'exemple n° 1).

On calcule le rendement en esters selon la relation précitée.

Les conditions opératoires mises en jeu dans cet exemple n° 12 conduisent à un rendement en esters de 67,3 %.

Une caractéristique importante de cette mise en oeuvre est l'élimination simultanée du glycérol par absorption sur le résidu solide.

## Revendications

1. Procédé de fabrication d'un ester d'alkyle, dans lequel on fait réagir un ester d'un alkyle différent et un alcool en présence d'un catalyseur acide à une température comprise entre 50° C et 250° C en vue de réaliser un échange de radicaux alkyles par une réaction de transestérification ou d'alcoolyse : ester d'alkyle1 + alcool2 $\Rightarrow$ ester d'alkyle2 + alcool1, ledit procédé étant caractérisé en ce que :

   .  on réalise la réaction en milieu mixte hydrophobe/hydrophile en présence d'un catalyseur acide comprenant essentiellement de l'acide phosphorique ou un sel de cet acide,
   .  on sépare par démixion la phase alcool hydrophile et la phase ester hydrophobe, et on sépare le cas échéant dans la phase ester l'ester d'alkyle 1 résiduel et l'ester d'alkyle 2 obtenu.

2. Procédé selon la revendication 1, dans lequel on réalise la réaction de transestérification ou d'alcoolyse à une température inférieure à la température d'ébullition du milieu réactionnel.

3. Procédé selon l'une des revendications 1 ou 2, caractérisé en ce qu'on utilise comme catalyseur de l'acide phosphorique dans de l'eau, en quantité telle que la proportion pondérale d'acide phosphorique par rapport à l'ester d'alkyle 1 soit sensiblement comprise entre 10 % et 50 % et que la proportion pondérale d'eau par rapport audit ester d'alkyle 1 soit sensiblement comprise entre 0,5 % et 5 %.

4. Procédé selon la revendication 3, dans lequel on utilise de l'acide phosphorique à 85 % dans l'eau.

5. Procédé selon l'une des revendications 1, 2, 3 ou 4, dans lequel on ajuste la proportion d'alcool par rapport à l'ester d'alkyle 1 dans une plage située sensiblement entre 2P et 4P, où P est la quantité d'alcool correspondant à la stoechiométrie de la réaction de transestérification ou d'alcoolyse.

6. Procédé selon la revendication 5, dans lequel on utilise un mono-alcool comme réactif.

7. Procédé selon l'une des revendications 1 à 6, dans lequel on utilise un alcool lourd à nombre de carbone supérieur ou égal à 8 et on ajuste la température du milieu réactionnel dans une plage comprise entre 120° et 170° C.

8. Procédé selon l'une des revendications 1 à 6, dans lequel on utilise un alcool court à nombre de carbone inférieur à 8 et on ajuste la température du milieu réactionnel dans une plage comprise entre 60° et 120° C.

9. Procédé selon l'une des revendications 1 à 8, dans lequel le milieu réactionnel est agité pour le disposer sous la forme d'une émulsion fine et stable.

10. Procédé selon l'une des revendications 1 à 9, dans lequel on réalise la démixion à froid par décantation de la phase hydrophile et on prélève la phase hydrophobe supérieure constituée de l'ester d'alkyle 2 obtenu, éventuellement d'ester alkyle 1 résiduel, et d'alcool résiduel.

11. Procédé selon la revendication 10, dans lequel on soumet la phase hydrophobe à une distillation fractionnée en vue de séparer l'alcool résiduel.

12. Procédé conforme à l'une des revendications 1 à 11 pour la fabrication d'un ester d'acide gras et d'alkyle, dans lequel on utilise comme ester d'alkyle 1 une huile essentiellement constituée de triglycérides en vue de réaliser l'échange des radicaux alkyles par déplacement du glycérol selon la réaction de transestérification complète :
triglycérides + alcool ⇒ ester d'acide gras + glycérol.

13. Procédé selon la revendication 12, dans lequel on utilise une huile brute provenant de la trituration de graines oléagineuses.

14. Procédé selon la revendication 12, dans lequel on utilise un broyat de graines oléagineuses provenant d'une action mécanique de broyage desdites graines.

15. Procédé selon l'une des revendications 12, 13 ou 14, dans lequel on soumet la phase hydrophile à une distillation fractionnée de façon à séparer le glycérol de l'alcool réactif résiduel en vue d'une valorisation du glycérol.

16. Procédé selon l'une des revendications 1 à 11 pour la fabrication d'un ester d'alkyle dans lequel le radical alkyle possède un nombre de carbone supérieur ou égal à 4, dans lequel on utilise un ester d'alkyle 1 ayant un radical alkyle à nombre de carbone inférieur à 4 en vue de réaliser l'échange des radicaux alkyles par une réaction d'alcoolyse équilibrée.

17. Procédé selon la revendication 16, dans lequel, après démixion, on sépare dans la phase hydrophobe l'ester d'alkyle à radical supérieur obtenu par distillation fractionnée ou extraction liquide/liquide dans des conditions propres à éliminer l'ester de départ résiduel.

Office européen
des brevets

**RAPPORT DE RECHERCHE EUROPEENNE**

Numéro de la demande

EP 99 39 0011

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | US 3 098 093 A (HUGH J. HAGEMEYER JR. ET AL.) 16 juillet 1963 (1963-07-16) * colonne 3, ligne 21 - ligne 30 * * colonne 8 - colonne 10; revendications *<br>--- | 1 | C07C67/03 C07C69/52 |
| A | WO 96 40701 A (THE PROCTER & GAMBLE COMPANY) 19 décembre 1996 (1996-12-19) * page 12, ligne 5 - page 15, ligne 2 * * page 19 - page 20; revendications *<br>----- | 1 | |

| | | | DOMAINES TECHNIQUES RECHERCHES (Int.Cl.6) |
|---|---|---|---|
| | | | C07C |

Le présent rapport a été établi pour toutes les revendications

| Lieu de la recherche | Date d'achèvement de la recherche | Examinateur |
|---|---|---|
| LA HAYE | 16 septembre 1999 | Kinzinger, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03 82 (P04C02)

**ANNEXE AU RAPPORT DE RECHERCHE EUROPEENNE
RELATIF A LA DEMANDE DE BREVET EUROPEEN NO.**

EP 99 39 0011

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-09-1999

| Document brevet cité au rapport de recherche | Date de publication | Membre(s) de la famille de brevet(s) | Date de publication |
|---|---|---|---|
| US 3098093 A | 16-07-1963 | AUCUN | |
| WO 9640701 A | 19-12-1996 | US 5648483 A | 15-07-1997 |
| | | AU 5935596 A | 30-12-1996 |
| | | BR 9609145 A | 17-02-1999 |
| | | CA 2223689 A | 19-12-1996 |
| | | CN 1190400 A | 12-08-1998 |
| | | EP 0848716 A | 24-06-1998 |
| | | NO 975543 A | 03-02-1998 |

EPO FORM P0460

Pour tout renseignement concernant cette annexe : voir Journal Officiel de l'Office europeen des brevets. No.12/82